(19) Europäisches Patentamt
European Patent Office
Office européen des brevets

(11) **EP 4 522 023 B1**

(12) **EUROPEAN PATENT SPECIFICATION**

(45) Date of publication and mention
of the grant of the patent:
**18.03.2026   Bulletin 2026/12**

(21) Application number: **23721967.0**

(22) Date of filing: **11.05.2023**

(51) International Patent Classification (IPC):
***A61B 5/11*** *(2006.01)*      ***A61B 5/00*** *(2006.01)*

(52) Cooperative Patent Classification (CPC):
**A61B 5/1124;** A61B 5/1121; A61B 5/6825;
A61B 5/6826

(86) International application number:
**PCT/EP2023/062682**

(87) International publication number:
**WO 2023/218002 (16.11.2023 Gazette 2023/46)**

(54) **FINGER DEXTERITY DEVICE**

FINGERFERTIGKEITSVORRICHTUNG

DISPOSITIF DE DEXTÉRITÉ DES DOIGTS

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority:   **13.05.2022   EP 22173373**

(43) Date of publication of application:
**19.03.2025   Bulletin 2025/12**

(73) Proprietor: **The Provost, Fellows, Foundation
Scholars and the
other members of Board, of the College of the
Holy
and Undivided Trinity of Queen Elizabeth,
Dublin 2 (IE)**

(72) Inventors:
• **HAYDEN, Conor**
  **Dublin (IE)**
• **MURRAY, Deirdre**
  **Dublin (IE)**
• **GERAGHTY, Dermot**
  **Dublin (IE)**

(74) Representative: **Barker Brettell LLP
100 Hagley Road
Edgbaston
Birmingham B16 8QQ (GB)**

(56) References cited:
WO-A1-2020/088017      WO-A1-99/21478
CN-A- 114 052 716      KR-A- 20210 118 606
US-A1- 2020 042 323      US-A1- 2020 260 994

**Description**

Technical Field

The present invention relates to a device and method for measuring motor function. More specifically, the invention relates to a device and method for measuring motor function for neurological practice by a finger tapping test.

Background to the Invention

Hand dexterity is an important outcome measure in most neurological conditions as loss of motor function is a common symptom in these conditions. This includes but is not limited to Parkinson's Disease, Multiple Sclerosis, Stroke, Cervical Myelopathy or other rarer neurological conditions that affect hand function.

Amyotrophic Lateral Sclerosis (ALS) (also known as Motor Neurone Disease (MND)) is a progressive and ultimately fatal neurodegenerative disease. The finger tapping test (FTT) is one of the most widely used measures of motor function in neurological practice. It involves tapping the index finger against the thumb rapidly while a clinician judges whether the movement is normal or abnormal by visually evaluating amplitude, frequency and accuracy. Performance grading has been utilised (for example) in the Movement Disorder Society Unified Parkinson's Disease Rating Scale (MDS - UPDRS) which incorporates a finger tapping task which rates participants on a 5-point scale. There are currently two main methods used to evaluate the FTT: tip of index finger to tip of thumb or tip of index finger to distal crease of thumb. The distal crease of the thumb method is generally considered the more sensitive measure. However, visual grading is subjective and insensitive to small but meaningful changes.

Additionally, some known methods involve mobile applications; for example, the participant taps a touchscreen of a computing device with the appropriate finger (sometimes with the hand placed flat on a surface) as high and as quickly as possible. Most, if not all, of the known mobile applications of this type are not validated for clinical use and focus on sports and rehabilitation, but some include different tests such as a two-target finger tapping test. A known digital platform for Parkinson disease patients has been developed by Apptomics. Originally known as iMotor (Apptomics Inc., Wellesley Hills, MA, USA), it is a mobile application that contains a two-target finger tapping test in which participants had to alternatingly tap the centre of two circles on a touchscreen with their index finger as fast and as accurately as possible.

Existing devices to measure and/or quantify dexterity using the FTT include that described in WO2016031348A1 to quantify bradykinesia scores in Parkinson's Disease. The device contains two magnetic coils (one detection, one generation of magnetic field) placed on the index finger and thumb. The distance between the two coils corresponds to a voltage due to electromagnetic induction. The voltage is then converted into a value by a nonlinear calibration model. Metrics such as maximum amplitude, total distance vs. frequency of taps and standard deviation of local max./min velocities in the opening/closing motion are extracted from the finger tapping motion. The device was tested on Parkinson Disease patients, whose data was then normalized according to age.

CN114052716A describes a wearable finger joint angle displacement data synchronous acquisition system. The system comprises a wearing unit, a data acquisition unit and a data processing unit. An angle sensor and a displacement sensor synchronously collect angle data of all joints and displacement data of corresponding fingers in a finger movement process of a patient, convert collected analogue signals into digital signals through an A/D converter and then transmit the digital signals to a STM32 single-chip microcomputer; then the STM32 single-chip microcomputer filters the transmitted data and then transmits the data to an upper computer through a wireless transmission module.

WO9921478A1 describes an exoskeleton device for measuring positions of links and angles of joints of an animate body, where the body comprises a plurality of links joined by intervening joints. The device is affixed at a first mobile terminus of said animate body, and a second fixed terminus, having device links displaced from animate links, where the device links are connected by device joints and having sensor means for measuring the angle of the device joints. Using the signals from the sensor means, one can determine the position of the terminal device link, and based on knowledge of the animate body structure calculate the animate angle joints.

WO2020088017A1 describes a hand robotic exoskeleton device, comprising: a main body part, a thumb mechanism, a fitting structure and an elastic mechanism, wherein the main body part can be used for wearing on four fingers and a palm; the thumb mechanism can be used for wearing on a thumb; the fitting structure can be fitted to the thumb, with one end of the fitting structure being connected to the main body part, and the other end thereof being connected to the thumb mechanism; and the elastic mechanism is arranged between the fitting structure and the main body part, and can be deformed when the thumb mechanism moves so as to apply a force to the fitting structure, so that the fitting structure is always in fitting contact with the thumb.

US2020042323A1 describes a technique for evaluating human cognitive and motor functions by a plurality of hand movement tasks. A task execution method determines the execution order of a plurality of tasks which a test subject is caused to execute to acquire a characteristic quantity. A test subject group task database includes scores given in advance and characteristic quantities obtained from a plurality of tasks stored as past data corresponding to each of a plurality of test subjects. In a storage device, a differentiation precision database for a case in which test subjects are divided into two groups by predetermined threshold value scores differentiated by the characteristic quantities, or an estimation precision database for a case in which a score is estimated using the characteristic quantity for a predetermined score value is prepared for each of the tasks on the basis of the test subject group task database.

KR20210118606A describes a three-dimensional finger movement measurement device comprising: a pair of base parts disposed to be in contact with an upper side of the finger; a pair of movement parts each movably disposed on the pair of base parts; a rotation part that connects the pair of movement parts, and is rotatably coupled to each of the pair of movement parts; and a sensor part that moves inside of the rotation part by relative rotation between the movement part and the rotation part, and measures an angle of rotation.

However, known devices have limitations relating to data processing, data output and ease of use. It is an aim of the present invention to address, or at least mitigate, drawbacks of the prior art.

<u>Summary of the Invention</u>

According to a first aspect of the invention, there is provided a device for measuring finger dexterity, comprising a thumb portion configured to be releasably secured to a thumb, a finger portion configured to be releasably secured to a finger (preferably the distal phalanx of a finger), wherein the finger portion and the thumb portion are directly connected by a flexible connector; a first rotatable element; a rotation sensor configured to sense rotation of the rotatable element, a microprocessor, wherein the microprocessor is in communication with the rotation sensor, and a transmitter configured for wireless communication with a computing device, wherein the rotatable element is configured to rotate when the finger portion and thumb portion move relative to each other, and wherein the microprocessor is configured to generate rotation data in response to rotation of the rotatable element, wherein the transmitter is configured to transmit the rotation data to the computing device.

The device facilitates digitisation of the FTT for use in neurological assessments by virtue of an advantageous measurement method. Unlike orientation sensors (accelerometer, gyroscope etc.), no filtering of the data is required - the data is clean and is immediately available for processing. The small footprint of the device also means that it is suitable for remote monitoring and is not hindered by a laborious set up procedure. No expertise is required for the operation of the device. The data is stored after every test completion based on input from the user or tester. The data storage process may also be automated regardless of input from the user or tester. The easy set-up also reduces error in the results - unlike IMU sensors, a small change in position or orientation will not affect the data output. The device can be zeroed before every test. The microcontroller and wireless capability remove any dependency on laptops and wired connections.

Preferably, the rotatable element is a magnet and wherein the rotation sensor is a magnetic rotary encoder. The inclusion of the magnetic rotary encoder means that there is no noise inherent in the device.

In the device according to the invention, one end of the flexible connector is affixed to the finger portion and the other end of the flexible connector is affixed to the thumb portion, and the flexible connector is configured to wind around a second rotatable element when the thumb portion and finger portion move towards each other. A spiral torsion spring is fixed to the second rotatable element, wherein the spiral torsion spring is arranged to contract when the thumb portion and the finger portion are moved away from each other.

Contraction of the spiral torsion spring causes rotation of the first rotatable element.

Preferably, the spiral torsion spring is housed in the thumb portion. Preferably, the microprocessor and transmitter are housed in a wrist portion, wherein the wrist portion comprises securing means for securing the wrist portion to a wrist.

The device preferably further comprises a timer configured to record the time required to complete a predetermined number of finger taps by a participant performing a finger tapping test.

According to a second aspect of the invention, there is provided a system comprising the device described herein and a computing device configured to receive time and rotation data, plot a graph of rotation data as a function of time and output the graph on a display of the computing device. Optionally, the graph is a plot of distance as a function of time.

The computing device is preferably configured to convert the magnet rotation data into distance data by division by a predetermined calibration factor. The computing device is further preferably configured to store data relating to the maximum finger extension of a participant. The computing device is optionally further configured to upload raw and/or processed test data to cloud storage.

The computing device may be further configured to calculate a score for each finger tapping test performed, wherein the score is a normalised height value divided by a normalised time value. The normalised height is preferably an average maximum extension distance between thumb and index finger recorded during a test divided by a maximum possible extension between thumb and index finger for a particular participant, and the normalised time is preferably the time to complete 10 finger taps divided by the fastest possible time to complete 10 finger taps. Preferably, the computing device is configured to upload raw and/or processed test data to cloud storage.

According to a third aspect of the invention, there is provided a method of measuring finger dexterity, comprising

generating, by a device described herein, time and rotation data pertaining to a finger tapping test, wirelessly transmitting the time and rotation data to a computing device, generating, by the computing device, a graph of rotation data as a function of time and outputting, by the computing device, the graph on a display.

Optionally, the method further comprises calculating, by the computing device, a score value for a finger tapping test using standardised data. Preferably, the method further comprises analysing the data, by the computing device, to determine at least one of average extension height, maximum extension height, time taken to complete a predetermined number of finger taps, number of hesitations and time taken to complete one tap motion.

According to a further aspect not covered by the present invention, there is provided a method of generating data relating to finger dexterity, comprising receiving time and rotation data from a finger dexterity device, wherein the time and rotation data relates to a finger tapping test performed using the finger dexterity device, converting the rotation data to distance data by multiplying by a calibration factor, retrieving a stored distance value and the stored time value, and calculating a score value based on the time data and distance data and retrieved stored time and stored distance values. The rotation data may be magnet rotation data.

Preferably, the stored distance value is the maximum finger extension of the participant who performed the finger tapping test. The stored time value may be fastest known time to complete the finger tapping test.

The method may further comprise generating a graph of distance as a function of time and displaying the graph on a display. Preferably, the finger dexterity device is a device described herein.

According to a yet further aspect not covered by the present invention, there is provided a method for calculating a dexterity performance score, comprising, by a data processing system comprising a machine learning model:

a) receiving a plurality of datasets derived from a plurality of finger tapping tests performed by a plurality of participants, wherein each dataset is derived from finger tapping test data performed by a participant using a finger dexterity device, wherein the plurality of participants includes a cohort of participants without a neurodegenerative condition and a cohort of participants with a neurodegenerative condition, and wherein each dataset comprises a plurality of metrics;

b) for at least one metric in the plurality of metrics, transforming values of the at least one metric in each dataset to provide a plurality of transformed datasets, wherein transforming comprises converting values to a normal distribution;

c) assigning either a first or second target variable to each transformed dataset, wherein the first target variable denotes that a dataset corresponds to a participant without a neurodegenerative condition and wherein the second target variable denotes that a dataset corresponds to a participant with a neurodegenerative condition,

d) generating a coefficient for each metric of the plurality of metrics based on the plurality of transformed datasets and the target variable for each dataset, and

e) calculating, using a prediction algorithm, a performance score for a transformed dataset using one or more transformed metrics and the respective coefficients corresponding to each metric, wherein the score is indicative of hand dexterity. Preferably, the machine learning model is a logistic regression model. Preferably, the coefficients are calculated based on their importance in determining at least the first target variable. Further preferably, the coefficients provide the change in the log-odds of the outcome for a one until increase in the targe variable.

According to a yet further aspect not covered by the present invention, there is provided a method for calculating a dexterity value, comprising, by a data processing system:

receiving dexterity data, wherein the dexterity data comprises a plurality of metrics relating to a finger tapping test performed using a finger dexterity device,

transforming values of at least one metric in the dataset to provide a transformed dataset, wherein transforming comprises converting a metric value to a normal distribution; and

calculating, by a machine learning model, a dexterity value based on the transformed dataset, wherein the dexterity value is indicative of hand dexterity. The dexterity value may be a probability value, and is preferably an objective score of hand dexterity. Preferably, the machine learning model is a trained model which uses co-efficient values (calculated for each metric) and the values of each metric as inputs to a prediction calculation. The prediction calculation may be characterised as the weighted sum of a constant and each metric multiplied by the corresponding coefficient.

Also provided is a computer readable medium storing executable instructions which, when executed by a processor, cause the method as described herein to be performed.

Brief description of the Figures

The invention will be described with reference to the following figures in which:

**Figures 1a and 1b** show the main components of a device according to an embodiment of the invention;
**Figures 2a and 2b** show opposing views of a device according to an embodiment of the invention attached to a thumb and forefinger;
**Figure 3** is an isometric view of a component of the device according to an embodiment of the invention;
**Figure 4** is an exploded view of the component of Figure 3;
**Figure 5** is an exploded view of a component of a device according to an embodiment of the invention;
**Figure 6a and 6b** are exploded and isometric views of a component of the device according to an embodiment of the invention;
**Figures 7a and 7b** are isometric views of a sub-component of the device according to an embodiment of the invention;
**Figure 8a and 8b** are exploded and isometric views of a sub-component of the device according to an embodiment of the invention;
**Figures 9a and 9b** are isometric views of a sub-component of the device of the device according to an embodiment of the invention;
**Figure 10** is a plan view of a sub-component of the device according to an embodiment of the invention;
**Figure 11** shows a PCB according to an embodiment of the invention;
**Figure 12** is a flow diagram of a method of generating finger dexterity data according to an embodiment of the invention;
**Figure 13** is a graph of exemplary processed data generated by a device according to an embodiment of the invention;
**Figure 14** is a graph of processed data generated by a device according to an embodiment of the invention;
**Figure 15** is a graph of processed data generated by a device according to an embodiment of the invention;
**Figure 16** is a graph of processed data generated by a device according to an embodiment of the invention;
**Figure 17** is a graph of processed data generated by a device according to an embodiment of the invention;
**Figure 18** is a graph of processed data generated by a device according to an embodiment of the invention;
**Figure 19** is a flow diagram of the main data processing steps to generate a performance score using a logistic regression model according to an aspect of the invention;
**Figure 20** shows the frequency distribution of performance scores in graphical form;
**Figure 21** shows dominant and non-dominant hand performance scores across age categories in graphical form; and

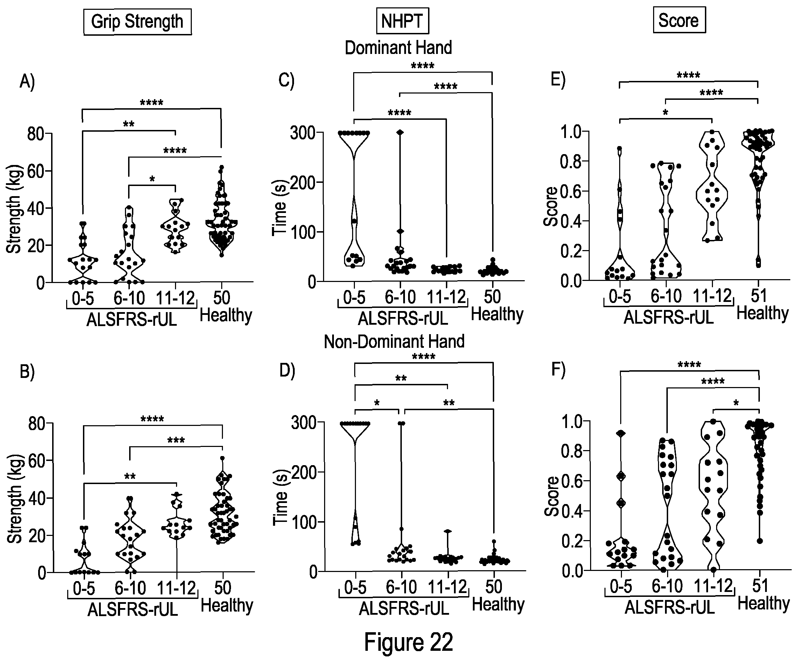

**Figure 22** shows dominant and non-dominant hand grip strength, NHPT and performance score in graphical form.

Detailed Description

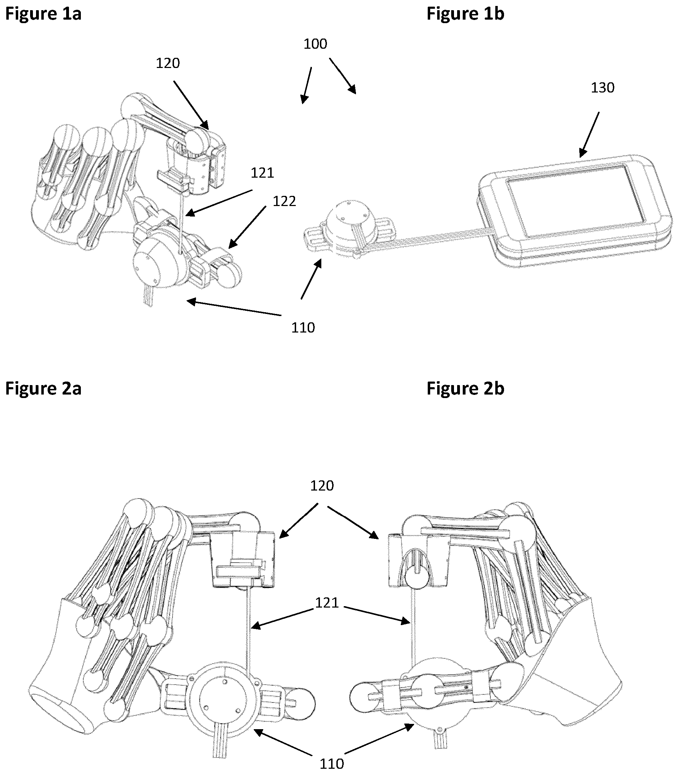

A finger dexterity device which digitises a finger tapping test is shown generally in Figures 1a and 1b. Device 100 comprises main body 110 which can be releasably secured to a participant's thumb with straps 122, which are preferably magnetic silicone straps. Straps 122 are adapted to enable attachment of many different thumb sizes and shapes. Finger attachment 120 is formed for attachment to the distal end (i.e. the distal phalanx) of a finger. For certain people and conditions being tested, it may be preferable for finger attachment 120 to be connected to an index finger, although the finger portion can be attached to any other finger (middle, ring or little/pinky). As shown in Figure 1b, main body 110 is physically connected to module 130.

Figure 2a and 2b further illustrate the arrangement of the device components affixed to a thumb and index finger of a right hand. Extension member 121 is an extendible chord that directly connects the main body 110 to finger attachment 120. Extension member 121 therefore extends between main body 110 and finger attachment 120 to provide a single link connection between a thumb and finger on one hand. As will be described below, the visible length of extension member 121 varies as a participant's index finger moves up and down (i.e., away from, and back towards, their thumb). The lengthening and shortening of extension member 121 causes rotation of a magnetic rotary encoder located within main body portion 110.

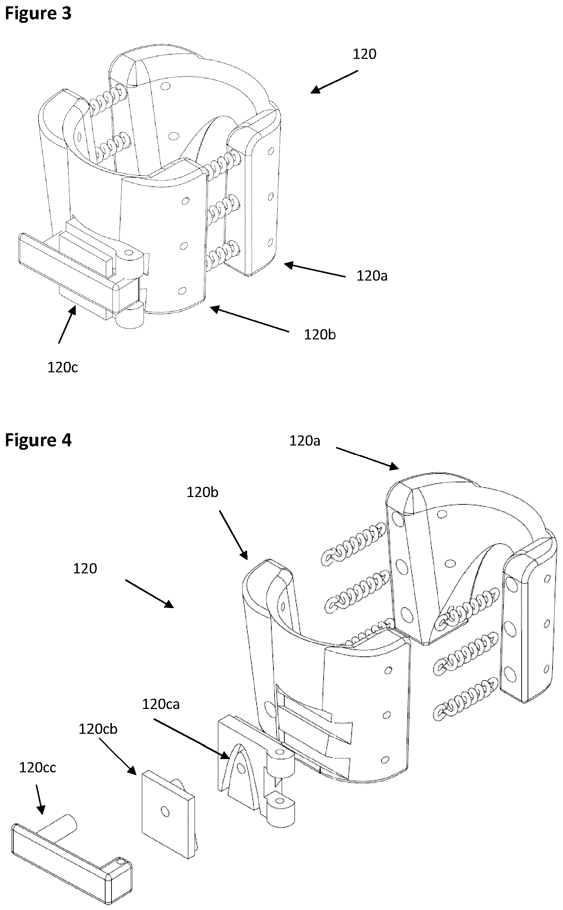

As shown in Figures 3 and 4, finger attachment 120 can be releasably secured to a participant's finger (e.g. the index finger), on the same hand on which the main body 110 is secured to the thumb. Finger attachment 120 is generally a two-part, spring-loaded component having an internal diameter than can expand to fit around an index finger and contract for securement. The spring-loaded mechanism allows finger attachment 120 to fit many different sized fingers.

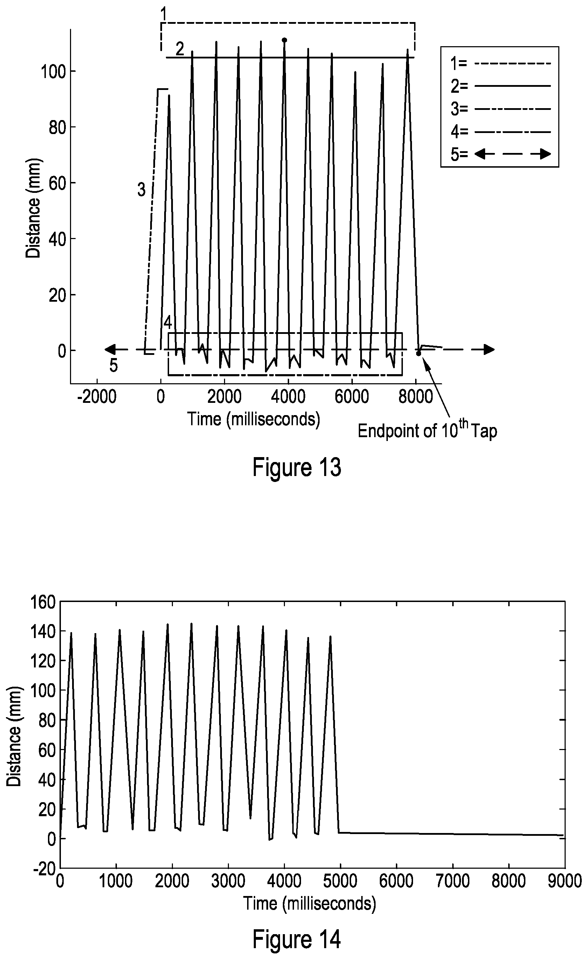

With reference to figure 4, finger attachment 120 generally comprises two opposing parts 120a, 120b connected by tension springs. Part 120a comprises a cut-out to allow a user to see their finger as they perform the test. Portion 120c (as shown in Figure 13) extends outwardly from portion 120b and provides means of detachable attachment of extension member 121. Portion 120c comprises at least one protrusion which fits within at least one groove on the outer surface of part 120b to create a dovetail joint. An end of extension member 121 fits into part 120ca and is secured in place by interference fit of part 120cb. A cylindrical protrusion on part 120cc is inserted into a corresponding hole or recess in part 120b to secure part 120c to part 120b.

As will be appreciated, parts 120a and 120b can be detached from part 120c whilst an end of extension member 121 is secured to portion 120c. As such, portions 120a and 120b can be changed (by, for example, replacement with similar parts with larger or smaller dimensions) to allow for securement to different fingers of differing sizes. Varying versions of portions 120a and 120b may have differing spring tensions to allow for greater or lesser extension and tension.

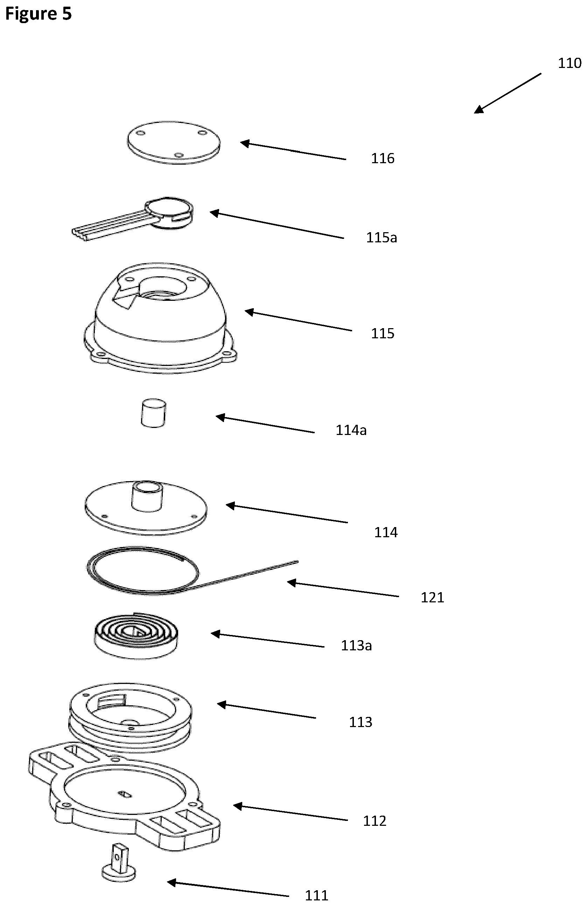

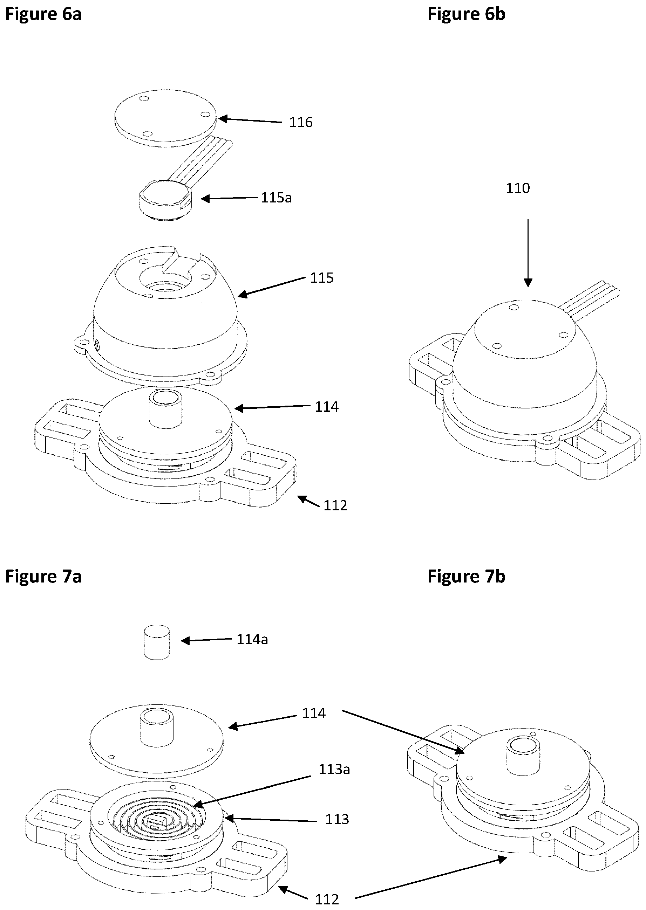

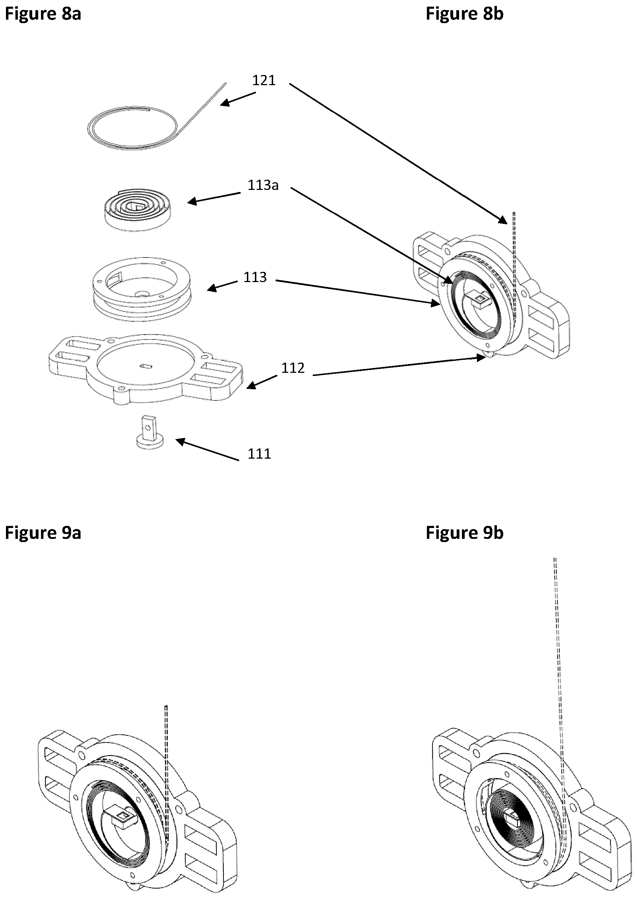

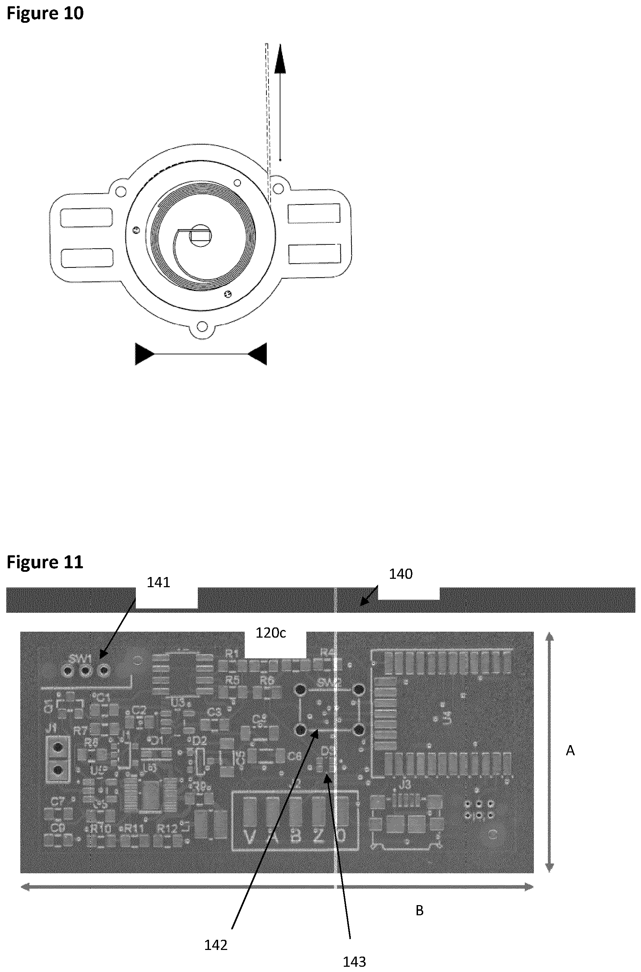

Main body portion 110 comprises multiple components, as shown generally in Figures 5, 6a and 6b. Base pin 111 fits within base 112. Base 112 comprises two opposing, laterally extending wings to facilitate attachment by attachment straps 122 and comprises a circular recess which is configured to receive and allow rotation of middle part 113 relative to base 112. Middle part 113 contains a hollow recess for receiving clock mainspring 113a. Clock mainspring 113a is affixed to middle part 113 such that clockwise rotation of middle part 113 causes clock mainspring 113a to wind/contract, and release/unwinding of the clock mainspring 113 causes counterclockwise rotation of middle part 113 (and thereby winding of extension member 121 around middle part 113). As illustrated in Figures 9a, 9b and 10, clock mainspring 113a contracts as middle part 113 rotates in a clockwise direction and reverts to its original position when middle part 113 rotates counterclockwise. Movement of the index finger away from the thumb causes extension member 121 to lengthen and unwind from the outer circumferential groove of middle part 113. This causes clock mainspring 113a to wind. As the participant moves their index finger towards their thumb, the tension in clock mainspring 113a is released, thereby causing extension member 121 to wind around the outer circumferential groove of middle part 113. The tension in clock mainspring 113a therefore ensures that extension member 121 winds around middle part 113 as the finger returns to the distal crease.

Container part 114 comprises a central cylindrical portion which is configured to house a 4x4mm cylindrical magnet 114a. Container part 114 is affixed to middle part 113 such that rotation of middle part 113 causes rotation of magnet 114a. Cover 115 fits over middle part 113 and container part 114 and is attached to base 112 with grub screws. Cover 115 comprises a hollow central chamber within which magnetic rotary encoder 115a is located. Lid 116 fits on the top of the cover 115 to secure magnetic rotary encoder 115a within cover 115.

In use, cover 115 and base 112 remain stationary as middle part 113 and container part 114 (within which magnet 114a is fixed) freely rotate clockwise and counterclockwise. Winding and unwinding of the clock mainspring causes clockwise and counterclockwise rotation of magnet 114a. The magnetic rotary encoder 115a detects rotation of magnet 114a located in container part 114 and 'counts' revolutions of magnet 114a. Pulses per revolution (ppr) refers to the number of periods on one of the quadrature signals in one revolution. Counts per revolution (cpr) is the number of changes of state on both channels of the encoder in one revolution and is achieved by electronically multiplying by 4, using the rising and falling edges of both channels of an encoder, i.e. cpr = 4 x ppr. The number of 'counts' per revolution depends on the resolution of the encoder - for example, a 5-bit encoder will record 32 pulses per revolution but will return a counts per revolution value of 8, i.e.; one revolution of the encoder = 8 counts. Similarly, a 12-bit encoder will record 4096 pulses per revolution and 1024 counts per revolution.

Magnetic rotary encoder 115a within main body portion 110 is connected to, and in communication with, module 130, as shown in Figure 1b. Module 130 is attached to a participant's wrist in use. Alternatively, module 130 can be placed in an appropriate location during the test that it does not interfere with the test or tester. Module 130 comprises a printed circuit board (PCB) on which a microcontroller, Bluetooth transceiver and rechargeable battery are fixed.

With reference to Figure 11, PCB 140 comprises connectors for on/off switch in location 141, push button in location 142 and LED in location 143. An LED is lit to denote whether magnetic rotary encoder 115a is counting (i.e whether a test is in progress). Width A is approximately 23mm and length B is approximately 53mm.

Module 130 is configured for selective wireless communication with a software application running on an external user device such as a smartphone, tablet or personal computer. The software application provides default control of device 100 via user interface features of the device in conjunction with the software application features. The test is preferably initiated from the software application. Initiation of a test begins a corresponding timer count in milliseconds. The counting is performed by a timer function of the microcontroller. In an alternative embodiment, counting is performed by a timer function of the software application, whereby the counting continues until the test is stopped via user input to the software application. A push button on module 130 may also be configured to act as a 'hard' stop for the counter in the event that the accompanying software used with device 100 fails to work. An alternative method for starting the test includes the push button on module 130. This may be used to 'zero' the test, such that when it is pushed, the magnetic encoder count is reset to zero. A corresponding timer also begins when the push button is depressed. The counts per revolution recorded by magnetic encoder 115a correspond to the number of revolutions of magnet 114a in a clockwise (or counterclockwise) direction, as discussed above.

Extension member 121 is wound around middle part 113 and an end of extension member 121 is secured to middle part 113. As a participant's thumb and finger move apart, extension member 121 is pulled out of the casing by unwinding from middle part 113. This causes mainspring 113a to wind/contract. In some embodiments, extension member 121 is elastic. Container part 114 rotates with middle part 113 such that when extension member 121 is extends and unwinds from middle part 113, magnet 114a also rotates (in a clockwise direction (or counterclockwise direction, depending on the configuration). This rotation is recorded by magnetic encoder 115a inserted in component 115. Magnetic encoder 115a 'counts' a predetermined number per complete revolution; the number of counts per revolution being dependent on the resolution of encoder 115a. In later processing, the number of counts allows the number of revolutions to be determined, which in turn allows for the distance moved to be determined (i.e. the distance moved by the index finger away from the thumb crease).

As the participant draws their index finger back towards their thumb crease, extension member 121 slackens, allowing mainspring 113a to release/unwind back to (or closer to) its original or starting tension. This causes middle part 113 to rotate counterclockwise which in turn causes magnet 114a to rotate counterclockwise. Encoder 115a records the number of counts as magnet 114a rotates counterclockwise. Encoder 115a records the count value at each turning point, i.e. when a change in direction is recorded. In later processing, the number of counts recorded during rotation in a counterclockwise direction by magnet 114a is used to determine the number of revolutions, and thereby the distance moved by the index finger, relative to the thumb crease, as it travels back towards the thumb crease. The clockwise and counterclockwise count data is recorded by the microcontroller, along with the corresponding time in milliseconds for each turning point. These turning points are then summed to get the total duration of the test.

The duration of the test and the clockwise and counterclockwise count number from the encoder are output by the microcontroller to the Bluetooth transmitter, which is preconfigured (i.e. configured before the start of the test) to transmit the time and count data to the external computing device for processing and storage. The ability to wireless transmit data via the Bluetooth transmitter advantageously means that the only wires required for the device to operate are those from the encoder within main body 120 to module 130. The data is preferably processed by the software application running on the external computing device. In a preferred embodiment, the data is sent by the computing device to a cloud server for pseudonymised or anonymised storage. The collection and storage of data from multiple test data for multiple participants allows for normalisation of the data, as described further below.

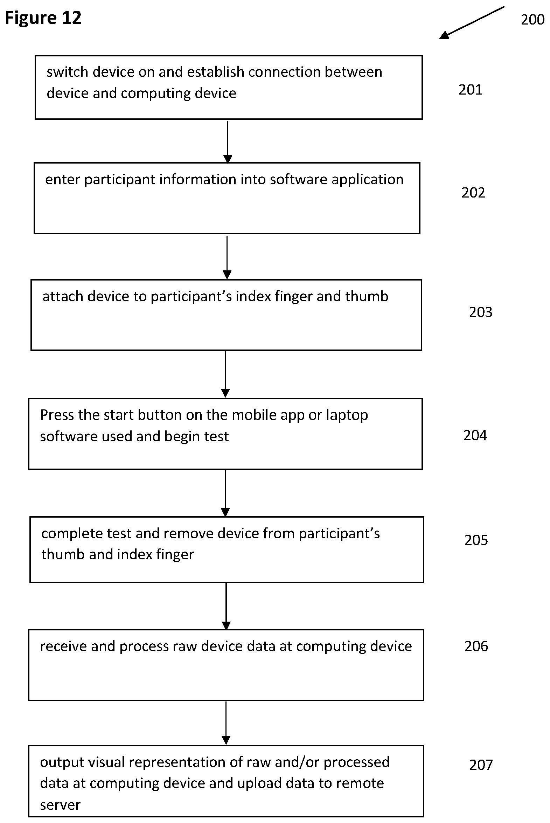

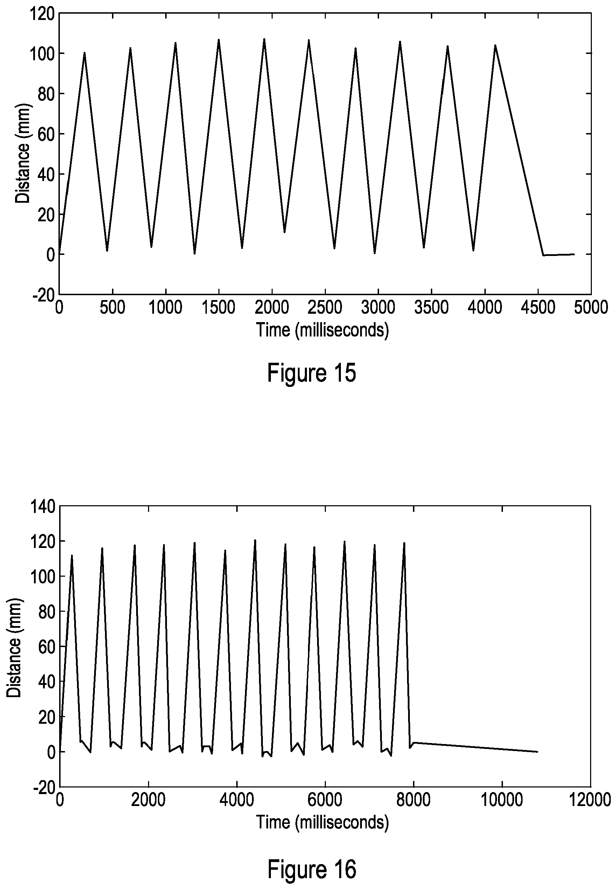

A process 200 of performing a test to generate the data is described with reference to Figure 12. At step 201, device 100 is switched on such that power from the battery is supplied to the components on the PCB and a Bluetooth connection is established between device 100 and an external computing device, according to mechanisms known in the art. At step 202, relevant information pertaining to the participant who will be performing the FTT is entered into a software application running on the computing device. For example, the participant may be a patient under clinical care, and may have a unique identification code. Preferably, age, gender, dominant hand and left/right hand test information is entered. At steps 203, 204 and 205, device 100 is attached to the participant's hand and the FTT test is performed. At step 206, the device data (i.e., the duration of the test and the counts for the clockwise and counterclockwise rotation phases) is transmitted to the computing device and processed to output, at step 207, a graphical representation of the test, as exemplarily shown in Figure 16. It will be appreciated that the order of the steps as outlined in Figure 15 depending on the particular implementation. Some steps may also overlap. For example, processing of the data at the computing device may be initiated upon receipt of the data from device 100 but whilst the device is still attached to a participant's finger. As a further example, a graphical representation of test data may be output after completion of one test whilst the device is attached to a participant's hand and before further test are started.

*Table 1*

| 1 | PhaseDuration | PulseCount |
|---|---|---|
| 2 | 0 | 0 |
| 3 | 33 | 0 |
| 4 | 0 | 1 |
| 5 | 218 | 1874 |
| 6 | 356 | 115 |
| 7 | 171 | 1721 |
| 8 | 325 | 77 |
| 9 | 183 | 1723 |
| 10 | 321 | 59 |
| 11 | 171 | 1753 |
| 12 | 371 | 62 |
| 13 | 162 | 1665 |
| 14 | 332 | 27 |
| 15 | 179 | 1672 |
| 16 | 115 | 90 |
| 17 | 183 | 89 |

(continued)

| 1 | PhaseDuration | PulseCount |
|---|---|---|
| 18 | 33 | 74 |
| 19 | 170 | 1676 |
| 20 | 109 | 59 |
| 21 | 205 | 58 |
| 22 | 22 | 84 |
| 23 | 175 | 1744 |
| 24 | 333 | 46 |
| 25 | 170 | 1724 |
| 26 | 121 | 44 |
| 27 | 197 | 44 |
| 28 | 31 | 51 |
| 29 | 158 | 1685 |
| 30 | 104 | 47 |
| 31 | 559 | 47 |
| 32 | 18 | 47 |
| 33 | 0 | 0 |
| 34 | 0 | 0 |

Table 1 is an exemplary raw data set output by the microcontroller. *'Phase Duration'* is the duration of each phase is milliseconds, where a 'phase' is the magnet rotating in a particular direction; *'Pulse Count'* is the number of rotations of the magnet during a particular phase. For example, the 4th phase (i.e., the 4th change of direction) lasted for 218ms and the encoder counted 1874 ('pulses').

The magnetic rotary encoder is preferably 12 bit, such that one full revolution of the magnet is equal to 1024 counts per revolution. The counts are recorded for the duration the magnet is in a particular state, i.e., rotating in a particular direction. The count is reset to zero and counting beings again by the encoder when the magnet changes state, i.e., it switches rotation direction. The interval between each of change of state of the magnet is recorded in milliseconds. This is seen in the corresponding pulse duration value for every pulse count. It can be seen from Table 1 that there are 10 'peaks' in the pulse count column. These peaks correspond to maximum index finger extension away from the thumb crease.

The pulse count values are converted to millimetres by dividing the count value by a calibration factor which is dependent upon each specific device. This calibration factor is calculated using a static testing machine or any other controlled displacement test (e.g., Zwick Instron etc). For example, a static machine extends the flexible member by a known amount. The distance is referenced against the pulse count value output from the device. A number of different measurements are taken a set number of times to determine the calibration value - i.e., what 1mm displacement corresponds to in pulse count value. For example, 1mm = 18.02 counts.

A raw data graph (for example, as shown in Figures 13-18) showing distance/time output of a finger tapping test using the device described herein is generated by summing the phase durations (x-axis) and plotting corresponding millimetre value based on conversion of the pulse count by the calibration factor (y-axis). The y-axis shows the distance between the thumb crease and the index finger, and the x-axis shows the time in milliseconds. The graph may be displayed on display apparatus of the external computing device or a separate display apparatus, such as a monitor.

The average extension height, maximim extension height and time to complete the test (for example, the time take to make 10 taps by the index finger at the thumb crease) can be determined from section 1 of the graph of Figure 13. The total number of extensions can be seen from section 2 of the graph, as well as the maximum distance of each extension. Section 4 of the graph (bottom rectangle) denotes the number of hesitations/time for one tap motion. The speed of each tap can be deduced from section 3 and the accuracy of each tap (i.e. how far the tap is away from the calibrated '0' position) can be deduced from a comparison with the horizontal line denoted by 5 on the graph. These metrics may be extracted/derived from the raw data by the microcontroller or the external computer device and output to a display interface. Table 2 summarises the metrics derivable from the raw data.

**Features**

| Height (mm) | • Average Extension Height<br>• Max Extension Height<br>When Passive Height Test Included:<br>• Average Extension Score<br>• Max Extension Score |
|---|---|
| Hesitation (ms) | • Average Hesitation Time<br>• Average Hesitation Height<br>• Average No. of Hesitations Events Per Test (max 9)<br>• Average No. of Hesitation Events Within Each Tap |
| Speed - Time (m/s) | • Time to Complete 10 taps<br>• Average Overall Speed of Taps<br>• Average Extension Speed of Taps<br>• Average Contraction Speed of Taps |
| Accuracy (mm) | • Average Distance Each Tap is away from start position (0mm) |
| Consistency (%) | • Change in tap height during the completed test (height of last tap divided by height of second tap) |

Plotting the raw data distance/time as per the example of Figure 13 provides near-instant, useful information which can be reviewed by a clinician to identify differences or changes compared to calibrated or previous participant test data. Such differences are easily recognisable from a comparison of Figures 14, 15 and 16. Figure 14 is an exemplary test output by a 20-year-old male on the right hand. Figure 15 is an exemplary output by a 40-year-old female on the left hand, and Figure 16 is a 70-year-old male on the right hand.

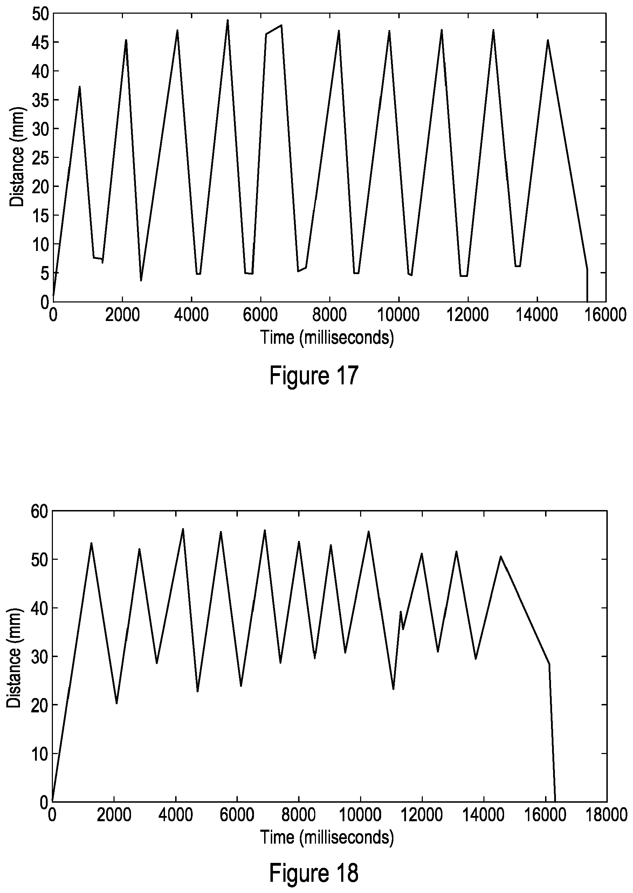

Most participants have a dominant hand (handedness) which is generally used for fine motor tasks. The differences in test output by the dominant vs non-dominant hand can be seen from a comparison of Figures 17 and 18. Figure 17 is a test output of participant 1 using the left, dominant hand. Figure 18 is a test output of participant 2 also using the left hand, but the left hand is non-dominant.

In preferred embodiments, the computing device not only outputs the raw test data in graphical form, but also calculates and outputs an overall score for each test as well as outputting values of average extension height, max extension height, time to complete the test, number of hesitations (and associated hesitation characteristics within each tap or over the test), time for one tap motion, speed of each tap, the accuracy of each tap and participant fatigue. In some embodiments, some metrics may be combined (e.g. accuracy and speed) to provide a meaningful data point. Any potential combination of factors may be useful. Normalisation techniques may be used on these values. A participant may perform a test multiple times (e.g., three times) to provide more accurate and statistically relevant data for clinical use. A 'passive' test (used to normalised for hand size) is performed on a participant's left and right hand to establish their maximum possible extension. This test is performed physically by the tester and recorded by the device as a separate data set. If one of the participant's tests is chosen as a best test for each hand, then a score is used to identify the test that should be used for such clinical or statistical purposes.

**Dominant Hand**

*Table 3*

| Dominant Hand | | | | | | |
|---|---|---|---|---|---|---|
| No. | AEH | TTC | PH | Normalised Height | Normalise Time | Overall Score |
| 013R1 | 113.86 | 8148.00 | 130.41 | 0.87 | 1.23 | 0.71 |
| 013R2 | 110.40 | 7111.00 | 130.41 | 0.85 | 1.07 | 0.79 |
| **013R3** | **117.05** | **6638.00** | **130.41** | **0.90** | **1.00** | **0.90** |

**Non-Dominant**

*Table 4*

| Non-Dominant | | | | | | |
|---|---|---|---|---|---|---|
| No. | AEH | TTC | PH | Normalised Height | Normalise Time | Overall Score |
| 013L1 | 103.16 | 7566.00 | 121.81 | 0.85 | 1.03 | 0.82 |
| **013L2** | **111.13** | **7329.00** | **121.81** | **0.91** | **1.00** | **0.91** |
| 013L3 | 109.03 | 7694.00 | 121.81 | 0.90 | 1.05 | 0.85 |

Tables 3 and 4 show relevant metrics for a healthy participant's right and left hand, where:

AEH = *Average Extension Height* (this is calculated by averaging the 10 peaks/extensions that correspond to a finger extension between thumb and index finger in a test)
TTC = *Time to Completion* (time in milliseconds to complete 10 taps)
PH = *Passive Height* (distance recorded when a tester/clinician extends the participant's thumb away from the index finger as far as possible)

*Normalised Height* (this is the AEH/PH and has a maximum possible value of 1. This can be converted to a percentage to show in real time what percentage of the passive height is reached)

*Normalised Time* (this is found by using the fastest TTC as the base level and dividing the 3 TTCs by this fasted TTC. The fastest time will always have a normalised time of 1. E.g., is the fastest TC is 6638, the normalised time in first row of table two = 8148/6638 = 1.23)

The overall score for the number of tests carried out for each hand per data collection set is calculated by dividing the normalised height by the normalised time. This is based on the test procedure whereby the participant extends their finger as high as they can, as fast as they can for 10 complete taps. The highest overall score can be manually or automatically identified and stored as pseudonymised or anonymised data for further statistical analysis, and/or used to identify the test to be used to make a clinical assessment. This overall score does not have to be used to find which of the tests is best. The data from all completed tests could be used for analysis.

Alternative methods of collecting hand dexterity data include the use of a motion control sensor or rotation sensor, such as a rotary encoder (absolute or incremental). Such a sensor converts rotational mechanical displacement into an electrical signal from which outputs such as speed and position are determined. Other types of rotational sensors that could be used to similar effect include hall effect sensors, potentiometers, optical encoders or rotary variable differential transformers (RVDT). This category of sensors also includes rotational speed sensors. These use various magnetic proximity principles to detect the speed of components. This family includes differential hall sensors, magneto inductive sensors, inductive sensors, oscillatory sensors, eddy current sensors or proximity switches. An infra-red (IR) sensor may also be used.

*Probability determination based on performance score*

As will be described in further detail below, a machine learning model is fit to a dataset which comprises the feature metrics (as per table 2 above) derived from the raw data of finger tapping tests for a number of people. Using a target variable (health/pwALS) the model provides coefficients for each metric. To generate a performance score for an individual, the feature metrics and corresponding coefficients are input to a prediction algorithm of the model which outputs a log-odds value. An exponential function is applied to the log-odds value to provide a performance score between 0 and 1. This performance score provides an indication as to whether the individual is healthy or is showing signs of a neurodegenerative condition. The closer the performance score to 0, the lesser the dexterity. The closer the performance score to 1, the greater the dexterity. The score may therefore be used as a measure of motor function. In this way, the score may also be characterized as a probability that a person has a condition affecting motor function.

The methodology described may be performed by suitable data processing apparatus. The ML model may be trained using a data processing system separate from a finger dexterity device and associated external computer. In an embodiment, the external computing device connected to the finger tapping device (as described above) has the necessary processing capabilities to process dexterity data from finger tapping tests using the trained ML model and output a performance score.

In the methodology described below, a logistic regression model is used. However, other machine learning models that return a single dexterity performance score may be used depending on the patient populations (Parkinson's Disease, Stroke, MS) and the score output may be specific to a particular condition. The particular model used may also depend on the quantity and quality of the data gathered.

The dataset for the training of the model comprised feature metrics derived from the raw data of finger tapping tests of two cohorts of participants: healthy people and people with Motor Neurone Disease/Amyotrophic Lateral Sclerosis (pwALS). Demographics, including sex, age, hand status and diagnosis (if required) were recorded. The healthy cohort included 180 participants (90 male, 90 female) comprising 30 males and 30 females in each of three age categories (20-39, 40-59, 60+). For inclusion, participants were required to be healthy and able to give informed consent. Participants were excluded if they had a diagnosis of a neurological condition or any condition affecting hand function. The pwALS cohort comprised 51 participants. The data from both cohorts were combined into one master dataset (n=231).

In order to compare the test performance of the pwALS to healthy performance, a subset of healthy people, age and sex matched to the pwALS, was derived from the healthy cohort for analysis. These people were chosen at random with a maximum age difference of $\pm$ 3 years. The relationship between two questionnaires (the general DASH (Disabilities of the Arm, Shoulder and Hand) and the ALSFRS-rUL (Revised Amyotrophic Lateral Sclerosis Functional Rating Scale)) on the pwALS dataset were examined using a Pearson's correlation coefficient. The ALSFRS-rUL results were divided into three score categories: normal function/mild disability (ALSFRS-rUL score: 11-12, n = 15), moderate disability (ALSFRS-rUL score: 6-10, n = 21) and severe disability (ALSFRS-rUL score: 0-5, n=14). Comparisons were carried out between these groups using a one-way ANOVA (with a Tukey's multiple comparison correction) or Kruskal-Wallis (with a Dunn's multiple comparison test) tests for parametric and non-parametric data respectively.

The test method for the dexterity device was standard for all participants. Firstly, they rested their hand in a custom hand support to standardise orientation. The tester attached the device to their thumb and index finger. Once the device was secured, they were instructed to place the tip of their index finger on the distal crease of their thumb and to extend their finger as high and as fast as they could, returning to the starting point of the distal crease between each tap, for eleven taps, ten of which were used for analysis. The two outputs from the test were distance travelled from the distal crease of the thumb and the corresponding time. Participants first completed a number of practice taps to ensure that the device was correctly secured. Three tests for both hands were completed. A 'best test' was chosen by identifying the test that met the criteria of shortest time and highest average extension height. A separate test, to record the maximal passive height of the movement was carried out once for each hand, which involved the tester passively extending the participant's index finger to the maximum potential height. Table 2 shows the 12 features that are extracted from a participant's raw data graph. An additional two features derived from the passive height test are also calculated. These features are the average and maximum height scores which return an output between 0-1, when the average and maximum extension height values are divided by the corresponding passive height value for a participant's hand. The dexterity device data was collected using a custom-built mobile application, which stored the raw data in a secure cloud storage, where it could be accessed for analysis.

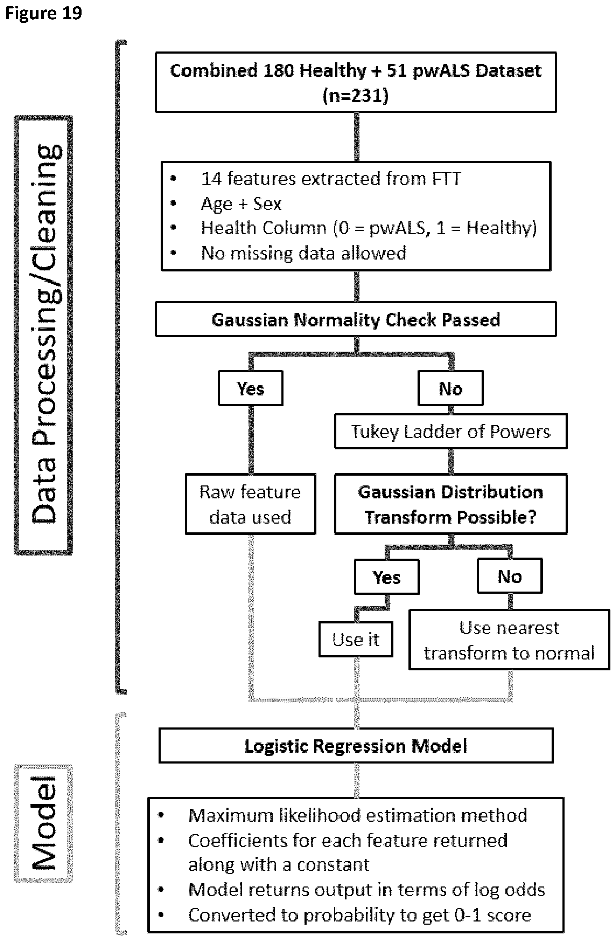

A logistic regression model, which used the maximum likelihood estimation method to find the model parameters, was fit to the combined dataset. Figure 19 provides a flow chart for this process. This method calculated the probability of observing the desired outcome variables given the input data and the model. It was optimised using the likelihood function (parameters that resulted in largest sum likelihood). The model used all 14 features extracted from the dexterity device raw data along with the participant's age and sex, which allowed these two effects to be accounted for in the model design. No missing data was allowed so if a participant was unable to use the dexterity device, the value for all the features were set to high values outside of a feasible performance range (i.e., 50000 seconds for time to completion or 500 millimetres or milliseconds for the hesitation features). This allowed these participants to be included in the model. A normality check was carried out and a Tukey Ladder of Powers test was carried out on all non-parametric features. Where possible, all features were transformed to follow a normal gaussian distribution. In the event a feature could not be normally transformed, the transform which was closest to a normal distribution was used (as shown in Table 5 below). This minimised the error in the model. A column indicating health status (Healthy = 1 / pwALS = 0) was added to the dataset. This health status column acted as the dependent/target variable in the regression model.

*Table 5: Distribution and transformation of the dexterity features for the logistic regression model in both dominant and non-dominant hands. A Tukey Ladder of Powers test was carried out on the 14 features to examine if they could be transformed to follow a normal distribution. If they could, that transform was used. If it was not possible, the transform which best approximated a normal distribution was used. The transformed used was noted.*

| Dexterity Features | Normal Gaussian Distribution? | | Logistic Regression Feature Transformation | |
|---|---|---|---|---|
| | D | ND | D | ND |
| Time to Completion (s) | Y | Y | Inverse | Inverse |
| Avg. Extension Height (mm) | Y | Y | Square | Cubic |
| Max Extension Height (mm) | N | Y | Square | Cubic |
| Avg. Extension Score | N | N | Cubic | Cubic |
| Max Extension Score | N | N | Cubic | Cubic |
| Avg. Hesitation Time (s) | N | N | Square root | Square root |
| Avg. Hesitation Height | N | N | Square root | Square root |
| Avg. No. of Hesitations per Test | N | N | Square root | Square root |
| Avg. No. of Hesitations per Tap | N | N | Square root | Square root |
| Avg. Accuracy (mm) | N | N | 1/ (Square root) | 1/ (Square root) |
| Absolute Consistency (%) | N | N | Square root | Square root |
| Avg. Extension Speed (m/s) | NA | NA | Identity | Identity |
| Avg. Contraction Speed (m/s) | N | N | Identity | Identity |
| Avg. Speed (m/s) | NA | NA | Identity | Identity |

*Y: Yes, the feature could be transformed to follow a normal distribution; N: No, the feature could not be transformed to follow a normal distribution; NA: Not applicable - feature was already normal; Identity: the non-transformed feature was used; D - dominant, NO - non-dominant.*

The output of the model was the predicted probability in log-odds units that a participant was healthy. This was calculated by assigning coefficients to each feature based on their importance in determining the dependent variable, along with a model constant. The logistic regression coefficients give the change in the log-odds of the outcome for a one unit increase in the predictor variable. The prediction equation (equation 1) for the model is the weighted sum of the constant and the features. This is also in units of log-odds. To obtain a continuous probability value, i.e., a performance score between 0-1 for all participants in the dataset, p, the probability of the event that a person is healthy was solved for (equation 2).

$$\log\left(\frac{p}{1-p}\right) = b_0 + b_1 * \text{Feature1} + b_2 * \text{Feature2} + b_3 * \text{Feature3} + \cdots \qquad (1)$$

where $b_0$ is a constant and $b_1, b_2, b_3$ .... are the coefficients applied to that particular feature. To solve for the probability, p, the exponential function is used.

$$p(\text{health}) = \frac{e^{(b_0 + b_1 * \text{Feature1} + b_2 * \text{Feature2} + b_3 * \text{Feature3} + \cdots)}}{1 + e^{(b_0 + b_1 * \text{Feature1} + b_2 * \text{Feature2} + b_3 * \text{Feature3} + \cdots)}} \qquad (2)$$

The values of these coefficients, along with other parameters estimated for the logistic regression model using the combined dataset (healthy participants and participants with ALS) when the target variable was health (1 = healthy, 0 = pwALS), are shown in Tables 6 and 7 for dominant and non-dominant hands respectively. The average number of hesitations per tap was not used in the model because of collinearity. The coefficients are used in equation 1 (*b0, b1, b2, b3,* etc.).

Table 6: The parameters estimated for the logistic regression model for dominant hand. The coefficients for the 16 features that were used to calculate the probabilities are noted along with the corresponding standard error, the z-value, the 2-tailed p-value used in testing the null hypothesis that the coefficient (parameter) is 0 and the 95% confidence interval for the coefficient.

| | Logistic Regression Model Dominant Hand | | | | | |
|---|---|---|---|---|---|---|
| | Coeff. | Std. err | z | P>z | [95% conf. interval] | |
| Constant | -3.142032 | 2.771943 | -1.13 | 0.26 | -8.57 | 2.29 |
| Age | -0.0142532 | 0.021119 | -0.67 | 0.50 | -0.06 | 0.03 |
| Sex | 1.061304 | 0.496297 | 2.14 | 0.03 | 0.09 | 2.03 |
| Time to Completion (ms) | 12280.34 | 16438.73 | 0.75 | 0.46 | -19938.98 | 44499.66 |
| Avg. Extension Height (mm) | 0.0015731 | 0.001267 | 1.24 | 0.22 | 0.00 | 0.00 |
| Max Extension Height (mm) | -0.0014066 | 0.001106 | -1.27 | 0.20 | 0.00 | 0.00 |
| Avg. Extension Score | -12.22249 | 13.77325 | -0.89 | 0.38 | -39.22 | 14.77 |
| Max Extension Score | 9.652522 | 11.14648 | 0.87 | 0.39 | -12.19 | 31.50 |
| Avg. Hesitation Time (ms) | 0.0966631 | 0.100334 | 0.96 | 0.34 | -0.10 | 0.29 |
| Avg. Hesitation Height | -0.3883815 | 0.5458 | -0.71 | 0.48 | -1.46 | 0.68 |
| Avg. No. of Hesitations per Test | -0.4191819 | 0.747653 | -0.56 | 0.58 | -1.88 | 1.05 |
| Avg. No. of Hesitations per Tap | 0 | (omitted) | | | | |
| Avg. Accuracy (mm) | -0.5129069 | 1.431098 | -0.36 | 0.72 | -3.32 | 2.29 |
| Absolute Consistency (%) | -0.0602828 | 0.168254 | -0.36 | 0.72 | -0.39 | 0.27 |
| Avg. Extension Speed (m/s) | 15.82697 | 9.023006 | 1.75 | 0.08 | -1.86 | 33.51 |
| Avg. Contraction Speed (m/s) | 0.2666364 | 9.61557 | 0.03 | 0.98 | -18.58 | 19.11 |
| Avg. Speed (m/s) | -5.717406 | 19.67377 | -0.29 | 0.77 | -44.28 | 32.84 |

Coeff: Coefficient; Std. err: Standard error; Avg.: average; z: z-score; s: seconds; mm: millimetre; %: percent; m/s: metres per second.

Table 7: The parameters estimated for the logistic regression model for non-dominant hand. The coefficients for the 16 features that were used to calculate the probabilities are noted along with the corresponding standard error, the z-value, the 2-tailed p-value used in testing the null hypothesis that the coefficient (parameter) is 0 and the 95% confidence interval for the coefficient.

| | Logistic Regression Model Non-Dominant Hand | | | | | |
|---|---|---|---|---|---|---|
| | Coeff. | Std. err | z | P>z | [95% conf. interval] | |
| Constant | -0.5514 | 2.246892 | -0.25 | 0.81 | -4.96 | 3.85 |
| Age (years) | -0.02739 | 0.018653 | -1.47 | 0.14 | -0.06 | 0.01 |
| Sex | 1.140125 | 0.510573 | 2.23 | 0.03 | 0.14 | 2.14 |
| Time to Completion (ms) | 15137.32 | 18927.23 | 0.80 | 0.42 | -21959.38 | 52234.01 |
| Avg. Extension Height (mm) | -3.96E-06 | 6.55E-06 | -0.60 | 0.55 | 0.00 | 0.00 |
| Max Extension Height (mm) | 4.45E-06 | 5.70E-06 | 0.78 | 0.43 | 0.00 | 0.00 |
| Avg. Extension Score | 5.272439 | 12.06915 | 0.44 | 0.66 | -18.38 | 28.93 |
| Max Extension Score | -5.02031 | 10.12801 | -0.50 | 0.62 | -24.87 | 14.83 |
| Avg. Hesitation Time (ms) | 0.063686 | 0.131614 | 0.48 | 0.63 | -0.19 | 0.32 |
| Avg. Hesitation Height (mm) | 0.504546 | 0.806777 | 0.63 | 0.53 | -1.08 | 2.09 |
| Avg. No. of Hesitations per Test | -0.3346 | 0.617646 | -0.54 | 0.59 | -1.55 | 0.88 |

(continued)

| | Logistic Regression Model Non-Dominant Hand | | | | | |
|---|---|---|---|---|---|---|
| | **Coeff.** | **Std. err** | **z** | **P>z** | **[95% conf. interval]** | |
| Avg. No. of Hesitations per Tap | 0 | (omitted) | | | | |
| Avg. Accuracy (mm) | -1.99612 | 1.219666 | -1.66 | 0.10 | -0.68 | 0.06 |
| Absolute Consistency (%) | -0.3116 | 0.188175 | -1.64 | 0.10 | -4.39 | 0.39 |
| Avg. Extension Speed (m/s) | 12.84409 | 7.967055 | 1.61 | 0.11 | -2.77 | 28.46 |
| Avg. Contraction Speed (m/s) | 3.603564 | 7.466539 | 0.48 | 0.63 | -11.03 | 18.24 |
| Avg. Speed (m/s) | -13.678 | 19.25056 | -0.71 | 0.48 | -51.41 | 24.05 |

*Coeff: Coefficient; Std. err: Standard error; Avg.: average; z: z-score; s: seconds; mm: millimetre; %: percent; m/s: metres per second.*

For each participant, an overall dexterity performance score was generated for the dexterity device for each hand and as an overall dexterity performance score representing both hands. The average dexterity performance score for all healthy participants (n=180) was $0.89 \pm 0.16 / 0.89 \pm 0.15$ (mean $\pm$ standard deviation) in the dominant and non-dominant hands respectively, compared with $0.82 \pm 0.20 / 0.82 \pm 0.20$ in the subset of healthy participants age and sex matched to the pwALS. This contrasted with pwALS scores of $0.38 \pm 0.32 / 0.40 \pm 0.32$ for the respective hands. The overall hand scores were: healthy participants = $0.90 \pm 0.11$, healthy subset = $0.82 \pm 0.17$ and $0.39 \pm 0.30$ for pwALS.

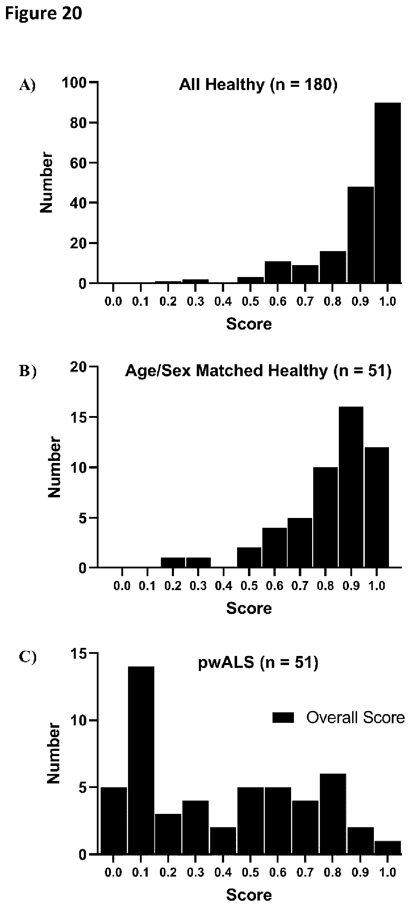

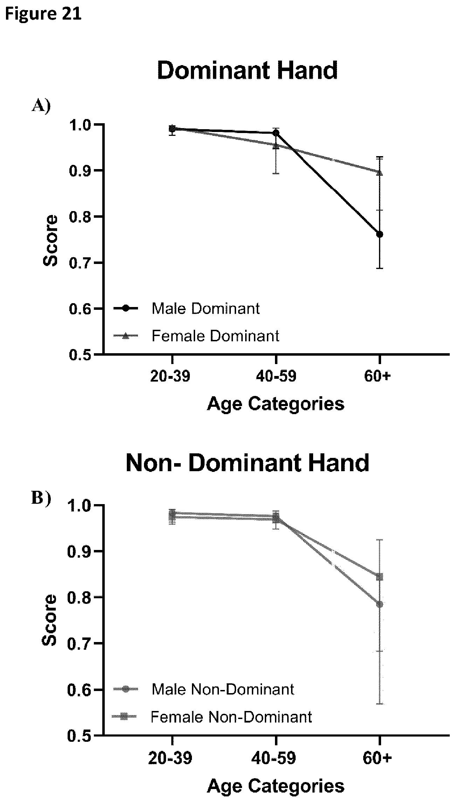

Figure 20 shows the change in distribution between the healthy participants, age/sex matched participants and pwALS for the overall dexterity performance score. Figure 21 highlights the change with age in the dexterity performance score for healthy participants, separated by sex and hand dominance. The median and 95% confidence interval are plotted for the three age categories in the healthy participants (separated by sex and hand dominance).

The dexterity performance score was moderately correlated with the traditional tests (Table 8). The grip strength test, NHPT (Nine-Hole Peg test) and dexterity performance score for the dominant and non-dominant hands were plotted against the three ALSFRS-rUL categories (mild, moderate and severe) and age/sex healthy subset (see Figure 21), which demonstrates the differences in test performance between these groupings. A Kruskal-Wallis was carried out between the groups. Significance is denoted by (*) using the convention $p < 0.05$ (*), $p < 0.01$ (**), $p < 0.001$ (***) and $p < 0.0001$ (****).

*Table 8: The correlation coefficients between the dexterity performance score, the two main traditional measures (grip strength and NHPT) and the two rating scales (DASH and ALSFRS-r) are presented for pwALS. The coefficients are calculated against the overall dexterity performance score and by dominant and non-dominant hand, where appropriate.*

| **Features** | **Overall** | |
|---|---|---|
| DASH | -0.51 | |
| ALSFRS-r | 0.45 | |
| ALSFRS-rUL | 0.49 | |
| | **Dominant** | **Non-Dominant** |
| Grip Strength | 0.38 | 0.52 |
| NHPT | -0.61 | -0.75 |
| DASH | -0.58 | -0.50 |
| ALSFRS-r | 0.44 | 0.51 |
| ALSFRS-rUL | 0.60 | 0.40 |

*Example of individual performance score calculation using trained model*

Table 9 shows 14 metrics extracted from a finger tapping test along with age and sex for two female participants, one healthy and one with the neurodegenerative condition, for both dominant and non-dominant hands. Prior to the calculation of a dexterity performance score, the feature metrics are transformed to follow as close to a normal distribution as possible (as discussed above).

*Table 9: Raw results for the two participants. The 14 finger tapping test features are shown along with age and sex. Results are shown for dominant and non-dominant hands respectively.*

| | Participant with neurodegenerative condition | | Healthy | |
|---|---|---|---|---|
| Age (years) | 69 | | 75 | |
| Sex | Female | | Female | |
| | **Dominant** | **Non-Dominant** | **Dominant** | **Non-Dominant** |
| Time to Completion (ms) | 7718 | 7610 | 7754 | 7567 |
| Avg. Extension Height (mm) | 101.5347 | 101.5897 | 107.0885 | 99.7598 |
| Max Extension Height (mm) | 113.6964 | 110.341 | 116.1101 | 107.6157 |
| Avg. Extension Score | 0.651571001 | 0.779941345 | 0.922301333 | 0.846371692 |
| Max Extension Score | 0.729615364 | 0.847128281 | 1 | 0.913021899 |
| Avg. Hesitation Time (ms) | 82.4444 | 0 | 176.3333 | 259 |
| Avg. Hesitation Height (mm) | 1.0696 | 0 | 1.2758 | 1.5101 |
| Avg. No. of Hesitations per Test | 3 | 0 | 4 | 7 |
| Avg. No. of Hesitations per Tap | 0.3333 | 0 | 0.6667 | 0.6667 |
| Avg. Accuracy (mm) | 3.1353 | 2.5908 | 4.2179 | 1.7223 |
| Absolute Consistency (%) | 12.8828 | 9.4301 | 8.0681 | 3.9658 |
| Avg. Extension Speed (m/s) | 0.3233 | 0.3143 | 0.435 | 0.3813 |
| Avg. Contraction Speed (m/s) | 0.213 | 0.2277 | 0.2123 | 0.2015 |

Avg. Speed (m/s) 0.2558 0.2613 0.2749 0.2601 *Coeff: Coefficient; Std. err: Standard error; Avg.: average; z: z-score; s: seconds; mm: millimetre; %: percent; m/s: metres per second.*

Table 10 shows the metric data after the transformations in Table 5 have been applied.

*Table 10: Results for the two participants after the transformations for the finger tapping test features have been applied. The 14 finger tapping test features are shown along with age and sex. Results are shown for dominant and non-dominant hands respectively.*

| | Participant with neurodegenerative condition | | Healthy | |
|---|---|---|---|---|
| Age (years) | 69 | | 75 | |
| Sex | Female | | Female | |
| | **Dominant** | **Non-Dominant** | **Dominant** | **Non-Dominant** |
| Time to Completion (ms) | 0.00013 | 0.000131406 | 0.000129 | 0.000132153 |
| Avg. Extension Height (mm) | 10309.295 | 1048453.161 | 0.000129 | 0.000132153 |
| Max Extension Height (mm) | 12926.87137 | 1343416.712 | 13481.55532 | 1246312.368 |
| Avg. Extension Score | 0.27662106 | 0.474444951 | 0.784546174 | 0.606294164 |
| Max Extension Score | 0.388402407 | 0.607921555 | 1 | 0.761103262 |
| Avg. Hesitation Time (ms) | 9.079889867 | 0 | 13.27905494 | 16.09347694 |
| Avg. Hesitation Height (mm) | 1.034214678 | 0 | 1.129513169 | 1.228861261 |
| Avg. No. of Hesitations per Test | 1.732050808 | 0 | 2 | 2.645751311 |
| Avg. No. of Hesitations per Tap | 0.577321401 | 0 | 0.816516993 | 0.816516993 |
| Avg. Accuracy (mm) | 0.5647555 | 0.621273824 | 0.4869136 | 0.761983556 |

(continued)

| | Participant with neurodegenerative condition | | Healthy | |
|---|---|---|---|---|
| Age (years) | 69 | | 75 | |
| Sex | Female | | Female | |
| | **Dominant** | **Non-Dominant** | **Dominant** | **Non-Dominant** |
| Absolute Consistency (%) | 3.589261763 | 3.070846789 | 2.840440107 | 1.991431646 |
| Avg. Extension Speed (m/s) | 0.3233 | 0.3143 | 0.435 | 0.3813 |
| Avg. Contraction Speed (m/s) | 0.213 | 0.2277 | 0.2123 | 0.2015 |

Avg. Speed (m/s) 0.2558 0.2613 0.2749 0.2601 *Coeff: Coefficient; Std. err: Standard error; Avg.: average; z: z-score; s: seconds; mm: millimetre; %: percent; m/s: metres per second.*

The coefficients from Tables 6 (dominant hand) and 7 (non-dominant hand) are presented together in Table 11. These are used as the inputs to equation 1.

*Table 11: The coefficients for the dominant and non-dominant hands used to calculate the dexterity performance score.*

| | **Dominant** | **Non-Dominant** |
|---|---|---|
| Constant | -3.142032 | -0.5514 |
| Age (years) | -0.0142532 | -0.02739 |
| Sex | 1.061304 | 1.140125 |
| Time to Completion (ms) | 12280.34 | 15137.32 |
| Avg. Extension Height (mm) | 0.0015731 | -3.96E-06 |
| Max Extension Height (mm) | -0.0014066 | 4.45E-06 |
| Avg. Extension Score | -12.22249 | 5.272439 |
| Max Extension Score | 9.652522 | -5.02031 |
| Avg. Hesitation Time (ms) | 0.0966631 | 0.063686 |
| Avg. Hesitation Height (mm) | -0.3883815 | 0.504546 |
| Avg. No. of Hesitations per Test | -0.4191819 | -0.3346 |
| Avg. No. of Hesitations per Tap | 0 | 0 |
| Avg. Accuracy (mm) | -0.5129069 | -1.99612 |
| Absolute Consistency (%) | -0.0602828 | -0.3116 |
| Avg. Extension Speed (m/s) | 15.82697 | 12.84409 |
| Avg. Contraction Speed (m/s) | 0.2666364 | 3.603564 |
| Avg. Speed (m/s) | -5.717406 | -13.678 |

As an example calculation for the 69 year old participant with a neurodegenerative condition, the log-odds value is calculated by multiplying the coefficient by its corresponding transformed feature (Table 10) e.g., the coefficient for time to completion for the dominant hand is 12280.34 while the transformed time to completion value is 0.00013. These are then multiplied by each other. This occurs for all the features along with an applied constant value, as per equation 1:

$$\log\left(\frac{p}{1-p}\right) = -3.142032 - (0.0142532 * 69) + (1.061304 * 1) + (12280.34 * 0.00013) +$$

$$(0.0015731 * 10309.295) - (0.0014066 * 12926.87137) - (12.22249 * 0.27662106) +$$

$$(9.652522 * 0.388402407) + (0.0966631 * 9.079889867) - (0.3883815 * 1.034214678) -$$

$$(0.4191819 * 1.732050808) + (0 * 0.577321401) - (0.5129069 * 0.5647555) - (0.0602828 *$$

$$3.589261763 + (15.82697 * 0.3233 + (0.2666364 * 0.213) - (5.717406 * 0.2558)$$

$$\log\left(\frac{p}{1-p}\right) = -0.11531029$$

The log-odds value is converted to a probability using equation 2. This probability is the dexterity performance score.

$$p(\text{health}) = \frac{e^{-0.11531029}}{1 + e^{-0.11531029}} = 0.471204327$$

The dexterity performance scores for the two participants are presented in Table 12.

Table 12: Dexterity Performance Score for the two participants. The dominant and non-dominant hand scores are shown.

|  | Participant with neurodegenerative condition | | Healthy | |
| --- | --- | --- | --- | --- |
| Age (years) | 69 | | 75 | |
| Sex | Female | | Female | |
|  | Dominant | Non-Dominant | Dominant | Non-Dominant |
| Dexterity Performance Score | 0.471204327 | 0.740868931 | 0.92619867 | 0.90110327 |

Claims

1. A device (100) for measuring finger dexterity, comprising

   a thumb portion (122) configured to be releasably secured to a thumb,
   a finger portion (120) configured to be releasably secured to the distal end of a finger,
   wherein the finger portion and the thumb portion are connected by a flexible connector (121), wherein one end of the flexible connector is affixed to the finger portion and the other end of the flexible connector is affixed to the thumb portion;
   a first rotatable element (114a);
   a rotation sensor (115a) configured to sense rotation of the first rotatable element,
   a microprocessor, wherein the microprocessor is in communication with the rotation sensor,
   a transmitter configured for wireless communication with a computing device,
   a second rotatable element (113), wherein the flexible connector is configured to wind around the second rotatable element when the thumb portion and finger portion move towards each other; and
   a spiral torsion spring (113a) fixed to the second rotatable element, wherein the spiral torsion spring is arranged to contract when the thumb portion and the finger portion are moved away from each other and wherein contraction of the spiral torsion spring causes rotation of the first rotatable element,
   and wherein the microprocessor is configured to generate rotation data in response to rotation of the rotatable element, wherein the transmitter is configured to transmit the rotation data to the computing device.

2. The device of claim 1, wherein the first rotatable element is a magnet and wherein the rotation sensor is a magnetic rotary encoder.

3. The device of any preceding claim, wherein the microprocessor and transmitter are housed in a wrist portion, wherein the wrist portion comprises securing means for securing the wrist portion to a wrist.

4. The device of any preceding claim, further comprising a timer configured to record the time required to complete a predetermined number of finger taps by a participant performing a finger tapping test.

5. A system comprising the device of any of claims 1 to 4 and a computing device configured to receive time and magnet rotation data, plot a graph of magnet rotation data as a function of time and output the graph on a display of the computing device.

6. The system of claim 5, wherein the computing device is configured to convert the magnet rotation data into distance data by division by a predetermined calibration factor.

7. The system of claim 5 or 6, wherein the computing device is further configured to store data relating to the maximum finger extension of a participant.

8. The system of claim 7, wherein the computing device is further configured to calculate a score for each finger tapping test performed, wherein the score is a normalised height value divided by a normalised time value.

9. The system of claim 8, wherein the normalised height is an average maximum extension distance between thumb and index finger recorded during a test divided by a maximum possible extension between thumb and index finger for a particular participant.

10. A method of measuring finger dexterity, comprising

generating, by a device according to claim 4, time and magnet rotation data pertaining to a finger tapping test, wirelessly transmitting the time and magnet rotation data to a computing device, generating, by the computing device, a graph of magnet rotation data as a function of time and outputting, by the computing device, the graph on a display.

11. The method of claim 10, further comprising calculating, by the computing device, a score value for a finger tapping test using standardised data.

12. The method of claim 10 or claim 11, further comprising analysing the data, by the computing device, to determine at least one of average extension height, maximum extension height, time taken to complete a predetermined number of finger taps, number of hesitations and time taken to complete one tap motion.

**Patentansprüche**

1. Vorrichtung (100) zum Messen der Fingerfertigkeit, umfassend

einen Daumenabschnitt (122), der so konfiguriert ist, dass er lösbar an einem Daumen befestigt werden kann, einen Fingerabschnitt (120), der so konfiguriert ist, dass er lösbar an dem distalen Ende eines Fingers befestigt werden kann, wobei der Fingerabschnitt und der Daumenabschnitt durch ein flexibles Verbindungsstück (121) verbunden sind, wobei ein Ende des flexiblen Verbindungsstücks an dem Fingerabschnitt befestigt ist und das andere Ende des flexiblen Verbindungsstücks an dem Daumenabschnitt befestigt ist; ein erstes rotierbares Element (114a); einen Rotationssensor (115a), der so konfiguriert ist, dass er die Rotation des ersten rotierbaren Elements erfasst, einen Mikroprozessor, wobei der Mikroprozessor mit dem Rotationssensor kommuniziert, einen Sender, der für die drahtlose Kommunikation mit einer Rechenvorrichtung konfiguriert ist, ein zweites rotierbares Element (113), wobei das flexible Verbindungsstück so eingerichtet ist, dass es sich um das zweite rotierbare Element wickelt, wenn sich der Daumenabschnitt und der Fingerabschnitt zueinander bewegen; und eine Spiraltorsionsfeder (113a), die an dem zweiten rotierbaren Element befestigt ist, wobei die Spiraltorsionsfeder so angeordnet ist, dass sie sich zusammenzieht, wenn der Daumenabschnitt und der Fingerabschnitt voneinander wegbewegt werden, und wobei das Zusammenziehen der Spiraltorsionsfeder eine Rotation des ersten rotierbaren Elements bewirkt,

und wobei der Mikroprozessor so konfiguriert ist, dass er Rotationsdaten als Reaktion auf eine Rotation des rotierbaren Elements erzeugt, wobei der Sender so konfiguriert ist, dass er die Rotationsdaten an die Rechenvorrichtung überträgt.

2. Vorrichtung nach Anspruch 1, wobei das erste rotierbare Element ein Magnet ist und wobei der Rotationssensor ein magnetischer Drehgeber ist.

3. Vorrichtung nach einem vorstehenden Anspruch, wobei der Mikroprozessor und der Sender in einem Handgelenkabschnitt untergebracht sind, wobei der Handgelenkabschnitt Sicherungsmittel zum Sichern des Handgelenkabschnitts an einem Handgelenk umfasst.

4. Vorrichtung nach einem vorstehenden Anspruch, weiter umfassend einen Timer, der so konfiguriert ist, dass er die Zeit aufzeichnet, die erforderlich ist, um eine vorbestimmte Anzahl von Fingertippen durch einen Teilnehmer, der einen Fingertippentest durchführt, abzuschließen.

5. System, das die Vorrichtung nach einem der Ansprüche 1 bis 4 und eine Rechenvorrichtung umfasst, die so konfiguriert ist, dass sie Zeit- und Magnetrotationsdaten empfängt, ein Diagramm der Magnetrotationsdaten als Funktion der Zeit erstellt und das Diagramm auf einem Display der Rechenvorrichtung ausgibt.

6. System nach Anspruch 5, wobei die Rechenvorrichtung so konfiguriert ist, dass sie die Magnetrotationsdaten durch Division durch einen vorbestimmten Kalibrierungsfaktor in Abstandsdaten umwandelt.

7. System nach Anspruch 5 oder 6, wobei die Rechenvorrichtung ferner so konfiguriert ist, dass sie Daten in Bezug auf die maximale Fingerstreckung eines Teilnehmers speichert.

8. System nach Anspruch 7, wobei die Rechenvorrichtung ferner so konfiguriert ist, dass sie einen Score für jeden durchgeführten Fingertippentest berechnet, wobei der Score ein normalisierter Höhenwert geteilt durch einen normalisierten Zeitwert ist.

9. System nach Anspruch 8, wobei die normalisierte Höhe ein durchschnittlicher maximaler Streckungsabstand zwischen Daumen und Zeigefinger ist, der während eines Tests aufgezeichnet wird, geteilt durch eine maximal mögliche Streckung zwischen Daumen und Zeigefinger für einen bestimmten Teilnehmer.

10. Verfahren zum Messen der Fingerfertigkeit, umfassend

Erzeugen, durch eine Vorrichtung nach Anspruch 4, von Zeit- und Magnetrotationsdaten im Zusammenhang mit einem Fingertippentest,

drahtloses Übertragen der Zeit- und Magnetrotationsdaten an eine Rechenvorrichtung,

Erzeugen, durch die Rechenvorrichtung, eines Diagramms der Magnetrotationsdaten als Funktion der Zeit und

Ausgeben des Diagramms durch die Rechenvorrichtung auf einem Display.

11. Verfahren nach Anspruch 10, weiter umfassend das Berechnen, durch die Rechenvorrichtung, eines Score-Werts für einen Fingertippentest unter Verwendung standardisierter Daten.

12. Verfahren nach Anspruch 10 oder Anspruch 11, weiter umfassend das Analysieren der Daten durch die Rechenvorrichtung, um mindestens eines von der durchschnittlichen Streckungshöhe, der maximalen Streckungshöhe, der Zeit, die benötigt wird, um eine vorbestimmte Anzahl von Fingertippen abzuschließen, der Anzahl der Zögerungen und der Zeit, die benötigt wird, um eine Tippbewegung abzuschließen, zu bestimmen.

**Revendications**

1. Dispositif (100) de mesure de la dextérité des doigts, comprenant

une partie de pouce (122) configurée pour être attachée de manière détachable à un pouce,

une partie de doigt (120) configurée pour être attachée de manière détachable à l'extrémité distale d'un doigt,

dans lequel la partie de doigt et la partie de pouce sont reliées par un connecteur flexible (121), dans lequel une extrémité du connecteur flexible est assujettie à la partie de doigt et l'autre extrémité du connecteur flexible est assujettie à la partie de pouce ;

un premier élément rotatif (114a) ;

un capteur de rotation (115a) configuré pour détecter la rotation du premier élément rotatif,

un microprocesseur, dans lequel le microprocesseur communique avec le capteur de rotation,

un émetteur configuré pour une communication sans fil avec un dispositif informatique,

un second élément rotatif (113), dans lequel le connecteur flexible est configuré pour s'enrouler autour du second élément rotatif lorsque la partie de pouce et la partie de doigt se déplacent l'une vers l'autre ; et

un ressort de torsion en spirale (113a) fixé au second élément rotatif, dans lequel le ressort de torsion en spirale est agencé pour se contracter lorsque la partie de pouce et la partie de doigt sont éloignées l'une de l'autre et dans lequel la contraction du ressort de torsion en spirale provoque la rotation du premier élément rotatif, et dans lequel le microprocesseur est configuré pour générer des données de rotation en réponse à la rotation de l'élément rotatif, dans lequel l'émetteur est configuré pour transmettre les données de rotation au dispositif informatique.

2. Dispositif selon la revendication 1, dans lequel le premier élément rotatif est un aimant et dans lequel le capteur de rotation est un codeur rotatif magnétique.

3. Dispositif selon une quelconque revendication précédente, dans lequel le microprocesseur et l'émetteur sont logés dans une partie de poignet, dans lequel la partie de poignet comprend des moyens d'attache pour attacher la partie de poignet à un poignet.

4. Dispositif selon une quelconque revendication précédente, comprenant en outre une minuterie configurée pour enregistrer le temps nécessaire pour effectuer un nombre prédéterminé de frappes au doigt par un participant réalisant un test de frappe au doigt.

5. Système comprenant le dispositif selon l'une quelconque des revendications 1 à 4 et un dispositif informatique configuré pour recevoir des données de temps et de rotation d'aimant, tracer un graphique de données de rotation d'aimant en fonction du temps et sortir le graphique sur un écran du dispositif informatique.

6. Système selon la revendication 5, dans lequel le dispositif informatique est configuré pour convertir les données de rotation d'aimant en données de distance par division par un facteur d'étalonnage prédéterminé.

7. Système selon la revendication 5 ou la revendication 6, dans lequel le dispositif informatique est en outre configuré pour stocker des données relatives à l'extension maximale de doigt d'un participant.

8. Système selon la revendication 7, dans lequel le dispositif informatique est en outre configuré pour calculer un score pour chaque test de frappe au doigt effectué, dans lequel le score est une valeur de hauteur normalisée divisée par une valeur de temps normalisée.

9. Système selon la revendication 8, dans lequel la hauteur normalisée est une distance d'extension maximale moyenne entre le pouce et l'index enregistrée pendant un test divisée par une extension maximale possible entre le pouce et l'index pour un participant particulier.

10. Procédé de mesure de la dextérité des doigts, comprenant

la génération, par un dispositif selon la revendication 4, de données de temps et de rotation de l'aimant relatives à un test de frappe au doigt,

la transmission sans fil des données de temps et de rotation d'aimant à un dispositif informatique,

la génération, par le dispositif informatique, d'un graphique de données de rotation d'aimant en fonction du temps et

la sortie, par le dispositif informatique, du graphique sur un écran.

11. Procédé selon la revendication 10, comprenant en outre le calcul, par le dispositif informatique, d'une valeur de score pour un test de frappe au doigt en utilisant des données standardisées.

12. Procédé selon la revendication 10 ou la revendication 11, comprenant en outre l'analyse des données, par le dispositif informatique, pour déterminer au moins l'une parmi la hauteur d'extension moyenne, la hauteur d'extension maximale, le temps nécessaire pour effectuer un nombre prédéterminé de frappes au doigt, le nombre d'hésitations et le temps nécessaire pour effectuer un mouvement de frappe.

**Figure 1a**

**Figure 1b**

100

120

121

122

110

130

**Figure 2a**

**Figure 2b**

120

121

110

EP 4 522 023 B1

**Figure 3**

120

120a

120b

120c

**Figure 4**

120a

120b

120

120ca

120cb

120cc

23

**Figure 5**

110

116

115a

115

114a

114

121

113a

113

112

111

**Figure 6a**                                    **Figure 6b**

116

115a

115

110

114

112

**Figure 7a**                                    **Figure 7b**

114a

114

113a

113

112

**Figure 8a**                                                          **Figure 8b**

121

113a

113

112

111

**Figure 9a**                                                          **Figure 9b**

EP 4 522 023 B1

**Figure 10**

**Figure 11**

## Figure 12

200

| switch device on and establish connection between device and computing device | 201 |

| enter participant information into software application | 202 |

| attach device to participant's index finger and thumb | 203 |

| Press the start button on the mobile app or laptop software used and begin test | 204 |

| complete test and remove device from participant's thumb and index finger | 205 |

| receive and process raw device data at computing device | 206 |

| output visual representation of raw and/or processed data at computing device and upload data to remote server | 207 |

Figure 13

Figure 14

Figure 15

Figure 16

Figure 17

Figure 18

**Figure 19**

**Figure 20**

**Figure 21**

## Dominant Hand

A)

## Non- Dominant Hand

B)

Figure 22

**EP 4 522 023 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2016031348 A1
- CN 114052716 A
- WO 9921478 A1
- WO 2020088017 A1
- US 2020042323 A1
- KR 20210118606 A